(19) Europäisches Patentamt
European Patent Office
Office européen des brevets

(11) Veröffentlichungsnummer: **0 009 658**
**B1**

(12) # EUROPÄISCHE PATENTSCHRIFT

(45) Veröffentlichungstag der Patentschrift: **26.08.81**

(51) Int. Cl.³: **C 07 C 55/06,** C 07 C 51/60

(21) Anmeldenummer: **79103333.5**

(22) Anmeldetag: **07.09.79**

(54) **Verfahren zur Herstellung von Oxalylchlorid.**

(30) Priorität: **16.09.78 DE 2840435**

(43) Veröffentlichungstag der Anmeldung: **16.04.80 Patentblatt 80/8**

(45) Bekanntmachung des Hinweises auf die Patenterteilung: **26.08.81 Patentblatt 81/34**

(84) Benannte Vertragsstaaten: **BE CH DE FR GB IT NL**

(56) Entgegenhaltungen:
**DE-B-1 220 856**
**DE-B-1 227 025**
**DE-B-1 245 958**

(73) Patentinhaber: **BAYER AG, Zentralbereich Patente, Marken und Lizenzen, D-5090 Leverkusen 1, Bayerwerk (DE)**

(72) Erfinder: **Vogel, Axel, Dr., In der Hildscheid 5, D-5068 Odenthal (DE)**
Erfinder: **Steffan, Guido, Dr., Herzogenfeld 52, D-5068 Odenthal (DE)**
Erfinder: **Mannes, Karl, Dr., Kurt-Schumacher-Ring 119, D-5090 Leverkusen (DE)**
Erfinder: **Trescher, Viktor, Dr., Völklinger Strasse 7, D-5090 Leverkusen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 009 658 B1

ACTORUM AG.

# Verfahren zur Herstellung von Oxalylchlorid

Die Erfindung betrifft ein Verfahren zur Herstellung von Oxalylchlorid aus Oxalsäureverbindungen, wie Oxalsäure oder Oxalsäureestern, und Phosphorpentachlorid in Gegenwart von Phosphoroxichlorid.

Nach den bekannten Verfahren zur Herstellung von Oxalylchlorid aus Oxalsäure durch Umsetzung mit Phosphorpentachlorid in Gegenwart von Phosphoroxichlorid (Ber. 41, 3558–3566, DRP 216918, DRP 216919, J. Am. Chem. Soc. 73, 4294–4296 (1951)) erreicht man Ausbeuten von nur 45 bis 55%. Bei der Herstellung von Oxalylchlorid nach diesen Verfahren sind lange Reaktionszeiten (18 bis 72 Stunden) erforderlich. Als Nebenprodukte fallen grosse Mengen Phosphoroxichlorid (2,6 bis 4,5 Mol pro Mol Oxalylchlorid), Chlorwasserstoff (etwa 4 Mol pro Mol Oxalylchlorid), sowie Kohlenmonoxid, Kohlendioxid und Phosgen an. Weiterhin werfen diese Verfahren schwierige sicherheitstechnische Probleme infolge der abrupt anspringenden und unter Ausstoss grosser Gasmengen verlaufenden Reaktion auf.

Aus der DE-AS 1220856, der DE-AS 1227025 und der DE-AS 1245958 ist ausserdem bekannt, Carbonsäurechloride durch Umsetzung von freien Carbonsäuren mit Phosphoroxichlorid bei erhöhter Temperatur in Gegenwart von tertiären organischen Stickstoffverbindungen und einer Verbindung der Alkali- oder Erdalkalimetalle oder in Gegenwart von primären oder sekundären organischen Stickstoffverbindungen oder deren Gemische oder in Gegenwart eines primären oder sekundären organischen Amins oder Carbonsäureamids herzustellen. Diese Chlorierungsverfahren lassen sich jedoch nicht auf die technische Herstellung von Oxalylchlorid übertragen, da sich Oxalsäure mit Phosphoroxichlorid nur in unbedeutenden Mengen zu Oxalylchlorid umsetzen lässt.

Es wurde ein Verfahren zur Herstellung von Oxalylchlorid aus Oxalsäureverbindungen der Formel

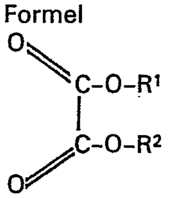

(I)

worin

$R^1$ und $R^2$ gleich oder verschieden sind und für Wasserstoff oder einen niederen Alkylrest stehen,

und Phosphorpentachlorid in Gegenwart von Phosphoroxichlorid gefunden, das dadurch gekennzeichnet ist, dass man die Umsetzung in Gegenwart einer Aminoverbindung der Formel

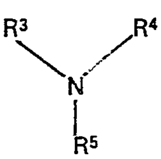

(II)

worin

$R^3$ für gegebenenfalls durch Amino- oder Carbonamid substituiertes Alkyl, Aralkyl, Aryl oder eine Acylgruppe steht und

$R^4$ und $R^5$ gleich oder verschieden sind und für Wasserstoff oder, gegebenenfalls durch Amino- oder Carbonamido substituiertes Alkyl, Aralkyl, oder Aryl stehen

oder

$R^4$ und $R^5$ in einem gegebenenfalls substituierten, gegebenenfalls Stickstoff, Schwefel und/oder Sauerstoff enthaltenden, und gegebenenfalls durch Alkyl-, Aralkyl-, Aryl- und/oder Aminogruppen substituierten carbocyclischen Ring mit 5 bis 7 Ringgliedern verbunden sein können und

$R^3$ für Wasserstoff, für gegebenenfalls durch eine Amino- oder eine Cabonamidogruppe substituiertes Alkyl, das mit $R^4$ zu einem carbocyclischen Ring verbunden sein kann, Aralkyl oder Aryl steht oder gegebenenfalls zu einem der Reste $R^4$ oder $R^5$ eine Doppelbindung bildet

gegebenenfalls in Gegenwart eines inerten Verdünnungsmittels durchführt und während der Reaktion das entstandene Oxalylchlorid abdestilliert.

Das erfindungsgemässe Verfahren kann durch die folgende Reaktionsgleichung erläutert werden:

$$2\ PCl_5 + HOOC\text{-}COOH \rightarrow ClCO\text{-}COCl + 2\ HCl + 2\ POCl_3$$

Niedere Alkylreste ($R^1$ und $R^2$) können geradkettige oder verzweigte Kohlenwasserstoffreste mit 1 bis 6, bevorzugt mit 1 bis 4, Kohlenstoffatomen sein. Beispielsweise seien Methyl, Äthyl, Propyl, Isopropyl, Butyl, Isobutyl, Pentyl, Isopentyl, Hexyl und Isohexyl, genannt.

Alkylreste ($R^3$, $R^4$, $R^5$) können geradkettige oder verzweigte Kohlenwasserstoffreste mit 1 bis 12, bevorzugt 1 bis 6, Kohlenstoffatomen sein. Beispielsweise seien genannt: Methyl, Äthyl, Propyl, Isopropyl, Butyl, Isobutyl, Pentyl, Isopentyl, Hexyl, Isohexyl, Heptyl, Isoheptyl, Octyl, Isooctyl, Nonyl, Isononyl, Decyl, Isodecyl, Undecyl, Isoundecyl, Dodecyl, und Isododecyl.

Aralkylreste ($R^3$, $R^4$, $R^5$) können Alkylreste mit 1–4 Kohlenstoff-Atomen, die durch einen aromatischen Kohlenwasserstoff-Rest mit 6–12 C-Atomen, bevorzugt Phenyl, Toluyl, substituiert sind, sein; beispielsweise Benzyl, o-, m-, p-Methylbenzyl. Arylreste ($R^3$, $R^4$, $R^5$) können aromatische Kohlenwasserstoffrest mit 6 bis 12 Kohlenstoffatomen, bevorzugt Phenyl, Toluyl sein.

Acylreste ($R^3$) können Reste der Formel

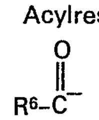

sein, worin $R^6$ ein geradkettiger oder verzweigter niederer Alkylrest, vorzugsweise mit 1 bis 6, insbesondere bevorzugt 1 bis 4, Kohlenstoffatomen,

oder ein aromatischer Rest, vorzugsweise ein Phenylrest, ist.

Beispielsweise sei genannt: Methyl, Äthyl, Propyl, Isopropyl, Butyl, Isobutyl, Pentyl, Isopentyl, Hexyl, Isohexyl und Phenyl.

Es ist auch möglich, dass der Rest $R^6$ mit einem der Reste $R^3$ oder $R^4$ zu einem ausser dem Aminstickstoff noch Kohlenwasserstoffglieder und gegebenenfalls Stickstoff, Schwefel und/oder Sauerstoff enthaltenden Ring, bevorzugt mit 5 oder 6 Ringgliedern, verbunden ist.

Beispielsweise sei der Pyrrolidonylring genannt.

Die Reste $R^4$ und $R^5$ können auch zu einem gegebenenfalls substituierten, (ausser dem Aminstickstoff gegebenenfalls Stickstoff, Schwefel und/oder Sauerstoff enthaltenden) carbocyclischen, bevorzugt aromatischen Ring, mit 5 bis 7, bevorzugt 5 oder 6, Ringgliedern gemäss Formel III, verbunden sein. Ausser dem Aminostickstoff kann dieser Ring gegebenenfalls auch 1 oder 2 Stickstoff-, Schwefel oder Sauerstoffatome enthalten. Der Ring kann mit 1-5 Alkyl-, Aralkyl-, Aryl- und/oder 1-2 Aminogruppen substituiert sein.

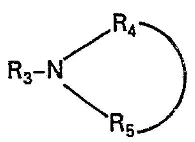

(III)

Beispielsweise seien als carbocyclische Ringe genannt: Pyrrolidin, Piperidin, Piperazin, Triazol, Pyrrol, Pyrazol, Imidazol, Thiazol, Oxazol, Morpholin, Pyridin, Pyrimidin, Triazin; diese carbocyclischen Ringe können weiterhin an kondensierte Benzoringe substituiert sein, beispielsweise Benzotriazol, Benzopyrrol, Benzoimidazol, Benzothiazol, Benzoxazol, Chinolin, Isochinolin, Indol, Acridin.

Die Reste $R^1$ bis $R^6$ können durch weitere Aminogruppen, bevorzugt 1 bis 2, oder Carbonamidogruppen, bevorzugt 1 bis 2, oder Alkyl-, bevorzugt 1 bis 2, Aralkyl-, bevorzugt 1-2, Aryl-, bevorzugt 1, substituiert sein. Die Substituenten $R^1$ bis $R^6$ können durch weitere Reste substituiert sein, die sich unter den Reaktionsbedingungen nicht verändern.

Beispielsweise seien genannt: die Halogene, bevorzugt Chlor und Brom, Nitro und Sulfonylgruppen. Als bevorzugte Substituenten der Verbindungen der Formel (III) seien die Dimethylaminogruppen und die Pyrrolidinoyl-Gruppe, insbesondere in para-Stellung zum Aminstickstoff, genannt sowie 1-2 Alkylgruppen mit 1-2 C-Atomen in p- und o-Stellung.

Bevorzugt für das erfindungsgemässe Verfahren sind Oxalsäureverbindungen der Formel IV

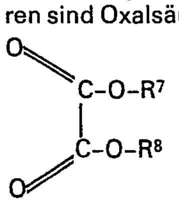

(IV)

worin

$R^7$ und $R^8$ gleich oder verschieden sind und Wasserstoff oder einen geradkettigen oder verzweigten Alkylrest mit 1 bis 4 Kohlenstoffatomen bedeuten.

Beispielsweise seien genannt: Oxalsäure, Oxalsäuredimethylester, Oxalsäurediäthylester, Oxalsäuredi-propylester, Oxalsäuredi-iso-propylester, Oxalsäuredi-n-butylester und Oxalsäuredi-iso-butylester. Bevorzugte Oxalsäureverbindungen sind Oxalsäure und der Oxalsäuredimethylester.

Oxalsäureverbindungen für das erfindungsgemässe Verfahren können nach bekannten Methoden (Ullmann, 3. Auflage, 13. Bd. S. 52) hergestellt werden, beispielsweise die Oxalsäure aus Natriumformiat durch Erhitzen auf 380°C unter Wasserstoffabspaltung und gegebenenfalls nachfolgend die Ester durch Umsetzung mit einem Alkohol.

Bevorzugte Aminoverbindungen für das erfindungsgemässe Verfahren sind tertiäre Amine und Carbonamide der Formel V

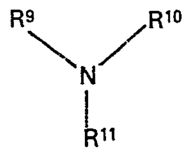

(V)

worin

$R^9$ für gegebenenfalls durch eine Amino- oder eine Carbonamidogruppe substituiertes Alkyl mit 1 bis 4 Kohlenstoffatomen, Aralkyl mit 7 bis 9 Kohlenstoff-Atomen, Phenyl oder Acyl mit 1 bis 8 Kohlenstoffatomen steht,

$R^{10}$ und $R^{11}$ gleich oder verschieden sind für gegebenenfalls durch Amino- oder Carbonamido substituiertes Alkyl mit 1 bis 4 Kohlenstoffatomen, Aralkyl mit 7 bis 9 Kohlenstoffatomen und Phenyl stehen, oder

$R^{10}$ und $R^{11}$ in einem 5- oder 6-gliedrigen Ring, der gegebenenfalls auch ein Stickstoff-, Schwefel- oder ein Sauerstoffatom als weiteres Heteroatom enthält und gegebenenfalls durch 1-5 Alkylgruppen mit 1-4 Kohlenstoffatomen, einen ankondensierten, gegebenenfalls weiter substituierten Benzolring oder 1-2 Dialkylamino- oder carbocyclischen Alkylidenaminogruppen mit je 1-4 Kohlenstoffatomen substituiert, verbunden sein können und

$R^9$ für Wasserstoff, für gegebenenfalls durch ein Amino- oder eine Carbonamidogrupep substituiertes Alkyl mit 1 bis 4 Kohlenstoffatomen das mit $R^{10}$ zu einem carbocyclischen Ring verbunden sein kann, Aralkyl mit 7 bis 9 Kohlenstoffatomen, Phenyl oder Acyl mit 1 bis 8 Kohlenstoffatomen steht oder gegebenenfalls zu einem der Reste $R^{10}$ und $R^{11}$ eine Doppelbindung bildet.

Als Aminoverbindungen für das erfindungsgemässe Verfahren seien beispielsweise genannt: Primäre, sekundäre und tertiäre Amine und Carbonsäureamide.

Insbesondere bevorzugt genannt seien die folgenden Aminoverbindungen:

a) tertiäre Amine der Formel

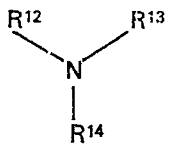

(VI)

worin $R^{12}$, $R^{13}$ und $R^{14}$ gleich oder verschieden sind und für gegebenenfalls substituiertes Alkyl mit 1 bis 4 Kohlenstoffatomen, Aralkyl mit 7 bis 9 Kohlenstoffatomen oder Phenyl stehen,

b) Carbonamide der Formel

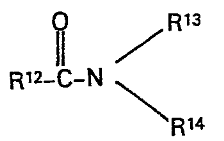

VII

worin $R^{12}$, $R^{13}$, $R^{14}$ die oben genannte Bedeutung haben und

c) heterocyclische Amine der Formel

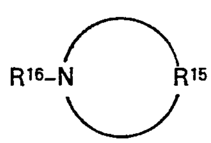

worin $R^{15}$ eine gegebenenfalls substituierte, aus Kohlenwasserstoffgliedern bestehende, gegebenenfalls noch ein weiteres Stickstoff-, Sauerstoff- oder Schwefelatom enthaltende, 4- oder 5-gliedrige Kette ist, und

$R^{16}$ für Wasserstoff, gegebenenfalls substituiertes Alkyl mit 1 bis 4 Kohlenstoffatomen, das mit $R^{16}$ zu einem carbocylischen Ring verbunden sein kann, Aralkyl mit 7 bis 9 Kohlenstoffatomen oder Phenyl steht oder eine Doppelbindung in dem aromatischen System bildet.

Als Amine seien beispielsweise genannt: Methylamin, Dimethylamin, Trimethylamin, Äthylamin, Diäthylamin, Triäthylamin, Propylamin, Dipropylamin, Tributylamin, N,N-Dimethylanilin, N,N-Diäthylanilin, N,N-Dipropylanilin, N,N-Dialkyltoluidine, Tribenzylamin, N,N-Dimethylbenzylamin, N,N-Diäthylbenzylamin, Äthylendiamin, N,N-Dimethyläthylendiamin, N,N'-Dimethyläthylendiamin, N,N,N',N'-Tetramethyläthylendiamin, Propylendiamin, Dimethylpropylendiamin, Tetramethylpropylendiamine, Diäthylentriamin, N,N',N''-Trimethyldiäthylentriamin, Permethyldiäthylentriamin, N,N-Dimethyl-neopentandiamin, N,N,N',N'-Tetramethyl-neopentandiamin, Pyrrolidin, 1-Methylpyrrolidin, Piperidin, 1-Methylpiperidin, 1-Äthylpiperidin, 1,2-Dimethylpiperidin, Piperazin, 1-Methylpiperazin, 1,4-Dimethylpiperazin, 1,2,4,5-Tetramethylpiperazin, 1,4,5,6-Tetrahydro-1,2-dimethylpyrimidin, 1,4-Diazabicyclo-[2,2,2]octan, 1,5-Diazabicyclo-[4,3,0]-non-5-en, 1,8-Diazabicyclo-[5,4,0]undec-7-en, Chinnuclidin, 1,3,5-Trimethylhexahydro-s-triazin, Hexamethyl-hexahydro-s-triazin, 1-Methyl-1,2,4-triazol, 1-Methylpyrrol, 1-1-Methyl-pyrazol, 1-Methyl-imidazol, 1,2-Dimethyl-imidazol, 1-Methylbenzimidazol, 1-Methyl-benzotriazol, Thiazol, 4,5-Dimethylthiazol, Benzothiazol, Oxazol, 2-Methyloxazol, 4,5-Dimethyloxazol, Benzoxazol, 2-Methylbenzoxazol, Morpholin, 1-Methylmorpholin, 4-Phenylmorpholin, 4,4'-(1,2-Äthandiyl)-bis-morpholin, Pyridin, 2-Methylpyridin, 3-Methylpyridin, 4-Methylpyridin, 2-Äthylpyridin, 3-Äthylpyridin, 4-Äthylpyridin, 2,4-Dimethylpyridin, 2,5-Dimethylpyridin, 2,6-Dimethylpyridin, 2-Äthyl-3-methylpyridin, 2-Äthyl-4-methylpyridin, 2-Äthyl-5-methylpyridin, 2-Äthyl-6-methylpyridin, 4-Äthyl-2-methylpyridin, 4-Äthyl-3-methylpyridin, 3-Äthyl-5-methylpyridin, 3-Äthyl-6-methylpyridin, 2,4,6-Trimethylpyridin und Isomere, 4-Dimethylamino-pyridin, 4-Pyrrolidinopyridin, 2,2'-Dipyridin, Chinoxalin, Pyrimidin, Methylpyrimidine, Dimethyl-pyrimidine, Triazin, Phenyltriazin, Trimethyltriazin, Thiazin, 1-Methyl-indol, Chinolin, Isochinolin, 2-Methylchinolin, 4-Methylchinolin, 6-Methylchinolin, 1-Methylisochinolin, 1-Methyl-1,2,3,4-tetrahydrochinolin, Acridin, Hexahydrotriamine, Pyrrol, Pyrazol, Imidazol, Triazol.

Bevorzugte Amine sind Trimethylamin, Triäthylamin, Pyridin, 2-Methylpyridin, 4-Methylpyridin, 4-Äthylpyridin, 2,4-Dimethylpyridin, 1,5-Diazabicyclo-[4,3,0]-non-5-en, 1,8-Diazabicyclo-[5,4,0]-undec-7-en, Dimethylpiperazin, Chinolin, Isochinolin.

Als Säureamide seien beispielsweise genannt Formamid, N,N-Dimethylformamid, Acetamid, N,N-Dimethylacetamid, N,N-Dimethylpropionamid, N,N-Dimethylbenzamid, N-Methylpyrrolidinon. Bevorzugte Säureamide sind N,N-Dimethylformamid, N,N-Dimethylacetamid.

Die Amine für das erfindungsgemässe Verfahren können nach bekannten Methoden hergestellt werden, beispielsweise durch Alkylierung von Ammoniak, Pyridin oder anderen heterocyclischen Aminen, die vorzugsweise aus Naturprodukten insbesondere Steinkohlenteer, isoliert werden, mit Alkylhalogeniden.

Die Carbonsäureamide für das erfindungsgemässe Verfahren können nach bekannten Methoden hergestellt werden, beispielsweise durch Umsetzung von Carbonsäurechloriden mit Ammoniak oder primären oder sekundären Aminen.

Für das erfindungsgemässe Verfahren ist es auch möglich, verschiedene Aminoverbindungen im Gemisch einzusetzen.

Die Einsatzformen der Amine und Carbonsäureamide für das erfindungsgemässe Verfahren sind beliebig variierbar. Im allgemeinen werden die Substanzen in reiner Form oder aber auch als Hydrochloride oder wässrige Lösungen zum Einsatz gebracht. Bei Verwendung wässriger Lösungen ist der Verbrauch an Phosphorpentachlorid durch Reaktion mit dem eingebrachten Wasser entsprechend zu berücksichtigen.

Für das erfindungsgemässe Verfahren ist es möglich, Phosphorpentachlorid sowohl in reiner als auch in technischer Form einzusetzen. Beispielsweise kann man Phosphorpentachlorid einsetzen, das man durch Umsetzung von Phosphortrichlorid mit Chlor erhält. (Ullman, 3. Aufl., 13 Bd., S. 562.)

Die Aminoverbindungen werden im allgemeinen in Mengen von 0,001 bis 0,5 Gewichtsteilen,

bevorzugt 0,005 bis 0,1 Gewichtsteilen, insbesondere bevorzugt 0,01 bis 0,05 Gewichtsteilen pro Gewichtsteil Phosphorpentachlorid, eingesetzt.

Die Umsetzung von Oxalsäure und Oxalsäureestern mit Phosphorpentachlorid nach dem erfindungsgemässen Verfahren wird in Gegenwart von Phosphoroxichlorid und gegebenenfals weiteren inerten Verdünnungsmitteln durchgeführt wie beispielsweise Methylenchlorid, Chloroform, Tetrachlorkohlenstoff, 1,2-Dichloräthan, 1,1,2,2-Tetrachloräthan oder 1,2-Dibromäthan. Im allgemeinen verwendet man 0,1–20 Gewichtsteile, bevorzugt 0,2–10 Gewichtsteile, insbesondere bevorzugt 0,3–2 Gewichtsteile pro Gewichtsteil Phosphorpentachlorid.

Die Umsetzung von Oxalsäure und Oxalsäureestern mit Phosphorpentachlorid nach dem erfindungsgemässen Verfahren wird im allgemeinen im Temperaturbereich von 10 bis 115 °C, vorzugsweise im Temperaturbereich von 20 bis 100 °C und insbesondere im Temperaturbereich von 40 bis 80 °C, durchgeführt. Im allgemeinen wird die Umsetzung bei einem Druck von 0,2 bis 2 bar, vorzugsweise 0,1 bis 1 bar und insbesondere bevorzugt von 0,15 bis 0,5 bar, durchgeführt.

Die Oxalsäure und Oxalsäureester werden in Substanz oder als Suspension oder als Lösung in Phosphoroxichlorid und gegebenenfalls in den anderen inerten, vorgenannten Verdünnungsmitteln eingesetzt. Bevorzugt wird Oxalsäure in Substanz oder als Suspension in Phosphoroxichlorid in einer Konzentration bis 1,5 Gew.-Teilen Oxalsäure pro Gew.-Teil Phosphoroxichlorid eingesetzt.

Für die stöchiometrische Umsetzung von Oxalsäure oder Oxalsäureestern zu Oxalylchlorid werden 2 Mol Phosphorpentachlorid benötigt. Nach dem erfindungsgemässen Verfahren ist es jedoch vorteilhaft Phosphorpentachlorid im Überschuss bis zu 20 Mol, bevorzugt bis zu 10 Mol, insbesondere bevorzugt bis zu 4 Mol, pro Mol Oxalsäure oder Oxalsäureester, einzusetzen. Das nicht umgesetzte Phosphorpentachlorid wird dabei im allgemeinen bei kontinuierlicher Arbeitsweise im Reaktor belassen oder bei diskontinuierlicher Arbeitsweise für eine weitere Umsetzung verwendet, so dass kein Verlust an Phosphorpentachlorid eintritt. Der Verbrauch an Phosphorpentachlorid beträgt dann mindestens 1,7 Mol, bevorzugt 1,8 bis 2,0 Mol, pro Mol Oxalsäure.

Das Phosporpentachlorid kann als Feststoff oder als Lösung oder als Suspension in Phosphoroxichlorid oder einem inerten Verdünnungsmittel eingesetzt werden. Im allgemeinen wird Phosphorpentachlorid nach bekannten Methoden (Ullman, 3. Auflage, 13. Bd., S. 562) durch Umsetzung von Phosphortrichlorid mit Chlor hergestellt.

In einer bevorzugten Ausführungsform des erfindungsgemässen Verfahrens wird zuerst das Phosphorpentachlorid und dann in dem gleichen Reaktionsmedium das Oxalylchlorid in einer sogenannten Eintopfreaktion hergestellt. Überraschenderweise gelingt die Herstellung von Phosphorpentachlorid aus Phosphortrichlorid und Chlor auch in Gegenwart der nach dem erfindungsgemässen Verfahren eingesetzten Amine und/oder Carbonsäureamide, ohne dass deren katalytische Aktivität durch die Reaktionsbedingungen der Phosphortrichlorid-Chlorierung verloren geht. Die Umsetzung von Phosphortrichlorid mit Chlor zu Phosphorpentachlorid wird in Gegenwart von Phosphoroxichlorid und gegebenenfalls inerten Verdünnungsmitteln und in Gegenwart der erfindungsgemäss verwendeten Amine und/oder Carbonamide durchgeführt. Dabei wird im allgemeinen im Temperaturbereich von 0 bis 130 °C, bevorzugt im Temperaturbereich von 20 bis 110 °C, und insbesondere bevorzugt im Temperaturbereich von 40 bis 90 °C gearbeitet. Der Druck beträgt dabei im allgemeinen 0,02 bis 2 bar, bevorzugt 0,1 bis 1,5 bar, insbesondere bevorzugt 0,15 bis 1 bar. Die Menge an Phosphoroxichlorid und gegebenenfalls inertem Verdünnungsmittel beträgt im allgemeinen 0,1 bis 20 Gewichtsteile, vorzugsweise 0,2 bis 10 Gewichtsteile, insbesondere bevorzugt 0,3 bis 2 Gewichtsteile, Phosphoroxichlorid und gegebenenfalls inertes Verdünnungsmittel pro Gewichtsteil erzeugtes Phosphorpentachlorid. Das zur Umsetzung benötigte Chlor wird im allgemeinen in einer Menge von mindestens 0,95 Mol, vorzugsweise 1 bis 1,1 Mol, pro Mol Phosphortrichlorid, eingesetzt.

Bei der erfindungsgemässen Umsetzung von Oxalsäureverbindungen mit Phosphorpentachlorid in Gegenwart von Aminoverbindungen wird das entstandene Oxalylchlorid während der Reaktion abdestilleirt. Vorzugsweise destilliert man das Oxalylchlorid im Gemisch mit Phosphoroxichlorid, wobei die Menge an mitdestilliertem Phosphoroxichlorid im Bereich 0–20 Gew.-Tl. Phosphoroxichlorid pro Gew.-Tl. Oxalylchlorid, bevorzugt 2–10 Gew.-Tl., eingestellt wird.

In der bevorzugten Ausführungsform des erfindungsgemässen Verfahrens destilliert man soweit Phosphoroxichlorid mit dem Oxalylchlorid ab, so dass im Reaktor die einmal vorgelegte Menge Phosphoroxichlorid bei mehreren Chargen der diskontinuierlichen Arbeitsweise hintereinander oder bei kontinuierlicher Arbeitsweise etwa konstant gehalten wird.

Das erfindungsgemässe Verfahren kann beispielsweise wie folgt durchgeführt werden:

Phosphorpentachlorid wird in Phosphoroxichlorid und gegebenenfalls in einem inerten Verdünnungsmittel in dem Reaktionsgefäss vorgelegt und dann die Oxalsäureverbindung, gegebenenfalls in Phosphoroxichlorid und gegebenenfalls in einem Vedünnungsmittel, hinzugegeben. Die Temperatur- und Druckbedingungen werden vorteilhafterweise so gewählt, dass das entstandene Oxalylchlorid während der Reaktion im Gemisch mit Phosphoroxichlorid abdestilliert. Das Oxalylchlorid wird hieraus anschliessend durch Rektifikation isoliert.

Falls man nach der bevorzugten Ausführungsform des erfindungsgemässen Verfahrens in einem Eintopfverfahren die Herstellung des Phosphorpentachlorids mit der Herstellung des

Oxalylchlorids verbindet, stellt man zuerst das Phosphorpentachlorid durch Umsetzung von Phosphortrichlorid mit Chlor, vorzugsweise in Phosphoroxichlorid als Lösungsmittel, in Gegenwart einer Aminoverbindung nach Formel II her. Ohne weitere Aufarbeitung kann man dann die Oxalsäureverbindung zugeben und erfindungsgemäss das Oxalylchlorid herstellen.

Nach dem erfindungsgemässen Verfahren erhält man vorteilhafterweise das Oxalylchlorid mit hohen Ausbeuten und grosser Reinheit. Sicherheitstechnisch bereitet das Verfahren keine besonderen Probleme.

Es ist überraschend, dass sich durch die erfindungsgemässe Arbeitsweise die Ausbeute an Oxalylchlorid verbessern lässt, obwohl die Bildung des Oxalylchlorids über die Stufe des äusserst instabilen Oxalsäure-monochlorids, dessen Isolierung selbst bei extrem tiefen Temperaturen noch nicht gelungen ist, und das sich sehr schnell zu Kohlenmonoxid, Kohlendioxid und Salzsäure irreversibel zersetzt, erfolgt (Ber. 46, 1426). Die bisher erhaltenen Ausbeuten betrugen daher nur maximal 55% der Theorie (Ber. 41, 3558, J. Amer. Chem. Soc 73, 4294). Dagegen werden nach dem erfindungsgemässen Verfahren Ausbeuten bis 95% der Theorie erzielt.

Gleichzeitig wird der Anfall an Nebenprodukten bis nahezu auf die stöchiometrische Menge Salzsäure reduziert. Oxalylchlorid ist ein Zwischenprodukt für Polyamide und Polyester (US 2816141).

Beispiel 1

In einem 2-l-Vierhalskolben mit Rührer, Feststoffdosiertrichter und Destillationsaufsatz werden 1000 g Phosphorpentachlorid und 12 g Triäthylamin in 500 g Phosphoroxichlorid suspendiert. Bei einer Reaktionstemperatur von 60°C und einem Arbeitsdruck von 250 mbar trägt man innerhalb 40 Minuten 100 g wasserfreie Oxalsäure ein. Zum Schluss rührt man 30 Minuten bei 70°C und 250 mbar nach. Während der Dosier- und Nachrührzeit fallen in der Destillationsvorlage 497 g eines Gemisches mit 20,7 Gew.-% Oxalylchlorid und 78,4 Gew.-% Phosphoroxichlorid an. In dem Reaktor verbleibt ein Gemisch, das 58 g nichtumgesetztes Phosphorpentachlorid und die Einsatzmenge Triäthylamin und restliches Phosphoroxichlorid enthält.

Die Ausbeute beträgt 73% der Theorie bezogen auf Oxalsäure bzw. 82% der Theorie auf Phosphorpentachlorid.

Beispiel 2

In das gemäss Beispiel 1 im Reaktor verbliebene Gemisch werden 412 g Phosphorpentachlorid und 75 g Phosphoroxichlorid nachgesetzt. Anschliessend trägt man bei einer Reaktionstemperatur von 60°C und einem Arbeitsdruck von 250 mbar 100 g wasserfreie Oxalsäure innerhalb 40 Minuten ein und rührt 30 Minuten bei 70°C/250 mbar nach.

Die Ausbeute in der Destillationsvorlage beträgt 513 g 20,3 Gew.-% Oxalylchlorid, entsprechend einer Ausbeute von 74% der Theorie bezogen auf Oxalsäure bzw. 83% der Theorie bezogen auf Phosphorpentachlorid.

Das im Reaktor verbliebene Gemisch enthält 588 g nichtumgesetztes Phosphorpentachlorid neben dem Einsatz Triäthylamin und kann nach Zusatz von Phosphorpentachlorid und Phosphoroxichlorid wie zuvor beschrieben erneut für die Umsetzung mit Oxalsäure verwendet werden.

Beispiel 3

In einer Apparatur gemäss Beispiel 1, werden 1000 g Phosphorpentachlorid, 12 g Triäthylamin und 500 g Phosphoroxichlorid bei 75°C vorgelegt. Unter Rühren trägt man bei einem Arbeitsdruck von 375 mbar 100 g Oxalsäure innerhalb 50 Minuten ein und rührt 30 Minuten bei 80°C/375 mbar nach.

Die Ausbeute beträgt 519 g 19,0 gew.-%iges Oxalylchlorid, entsprechend 70% der Theorie bezogen auf Oxalsäure.

Beispiele 4–16

In einer Apparatur gemäss Beispiel 1 werden gemäss Tabelle 1 1000 g Phosphorpentachlorid, a) g des unter b) genannten Amins und 500 g Phosphoroxichlorid bei einer Temperatur von c) °C und einem Arbeitsdruck von d) mbar vorgelegt. Unter Rühren trägt man 100 g wasserfreie Oxalsäure in e) Minuten ein und rührt 30 Minuten bei einer Temperatur von f) °C und einem Druck von d) mbar nach.

Die Ausbeute beträgt g) g Oxalylchlorid im Gemisch mit vorwiegend Phosphoroxichlorid. Die molare Ausbeute beträgt i) % der Theorie bezogen auf Oxalsäure.

| Beisp. Nr. | a | b | °C c | mbar d | Min e | °C f | g g | % h |
|---|---|---|---|---|---|---|---|---|
| 4 | 6 | 4-Äthylpyridin | 65 | 255 | 40 | 70 | 108 | 77 |
| 5 | 24 | 4-Äthylpyridin | 58 | 250 | 35 | 65 | 122 | 86 |
| 6 | 13 | Bis-(2-dimethylamino-äthyl)-methylamin | 70 | 260 | 40 | 75 | 89 | 63 |
| 7 | 20 | 4-Methylpyridin | 60 | 250 | 40 | 70 | 123 | 87 |
| 8 | 6 | 2,4-Dimethylpyridin | 60 | 250 | 30 | 70 | 120 | 85 |
| 9 | 35 | 2,4-Dimethylpyridin | 60 | 250 | 30 | 70 | 128 | 91 |
| 10 | 4 | Dimethylformamid | 60 | 250 | 30 | 70 | 115 | 82 |
| 11 | 6 | 1,4-Diazabicyclo [2,2,2]-octan | 60 | 250 | 30 | 70 | 87 | 62 |

| Beisp. Nr. | a | b | °C c | mbar d | Min e | °C f | g g | % h |
|---|---|---|---|---|---|---|---|---|
| 12 | 6 | 1,4-Dimethylpiperazin | 60 | 250 | 35 | 70 | 99 | 70 |
| 13 | 7 | 1,5-Diazabicyclo [4,3,0]-non-5-en | 60 | 250 | 35 | 70 | 112 | 79 |
| 14 | 7 | Chinolin | 60 | 250 | 40 | 70 | 98 | 69 |
| 15 | 7 | Isochinolin | 60 | 250 | 40 | 70 | 99 | 70 |
| 16 | 7 | N,N,2,2-Tetramethyl-propan-1,3-diamin | 60 | 250 | 45 | 70 | 99 | 70 |
| 17 | 12 | Triäthylamin | 45 | 150 | 40 | 50 | 98 | 70 |

Beispiel 18

In einer kontinuierlich arbeitenden Apparatur, bestehend aus einem Rührkessel-Reaktor mit Destillationsaufsatz sowie Mess- und Dosiergeräten für Oxalsäure-Phosphoroxichlorid- und Phosphorpentachlorid - Phosphoroxichlorid - Gemische, werden im Reaktor ein Gemisch aus 2000 Tl. Phosphorpentachlorid und 49 Tl. 2,4-Dimethylpyridin in 1000 Tl. Phosphoroxichlorid bei 60°C vorgelegt. Bei einem Arbeitsdruck von 250 mbar dosiert man aus einer gerührten Messvorlage stündlich eine Suspension von 307 Tl. Phosphorpentachlorid in 307 Tl. Phosphoroxichlorid und aus einer zweiten Messvorlage stündlich eine Suspension von 70 Tl. wasserfreie Oxalsäure in 70 Tl. Phosphoroxichlorid in den Reaktor ein. Über den Destillationsaufsatz destillieren stündlich 380 Tl. einer Mischung mit 23,5 Gew.-% Oxalylchlorid und 75,5 Gew.-% Phosphoroxichlorid ab. Das Oxalylchlorid wird hieraus durch fraktionierte Destillation über eine Kolonne isoliert. Die Ausbeute beträgt stündlich 89 Tl. Oxalylchlorid.

Beispiel 19

In einem 4 l Vierhalskolben mit Rührer, Gaseinleitrohr, und Destillationsaufsatz werden 1340 g Phosphoroxichlorid, 65 g 2,4-Dimethylpyridin und 1769 g Phosphortrichlorid vorgelegt. Dann leitet man bei einer Sumpftemperatur von 85°C innerhalb von 6 Stunden 930 g Chlor ein. Man destiliert die entstandene Phosphorpentachlorid-Suspension im Vakuum bis zur Entfernung des Chlorüberschusses an, kühlt auf 60°C ab und trägt im Verlauf von 4 Stunden bei 60°C Reaktionstemperatur und 250 mbar Arbeitsdruck eine Suspension von 375 g Oxalsäure in 375 g Phosphoroxichlorid ein. Zum Schluss rührt man den Ansatz $^1/_2$ Stunde bei 70°C/250 mbar nach. Während der Oxalsäure-Dosierung und der Nachrührzeit fallen hinter dem Destillationsaufsatz 2052 g Rohdestillat mit einem Gehalt von 21,9 Gew.-% Oxalylchlorid an. Durch fraktionierte Destillation werden hieraus 445 g Oxalylchlorid isoliert.

Beispiel 20

Die gemäss Beispiel 19 im Reaktor verbliebene Suspension wird mit 1025 g Phosphortrichlorid versetzt und anschliessend bei einer Sumpftemperatur von 85°C unter Rückflussbedingung innerhalb von 3 Stunden durch Einleiten von 570 g Chlor chloriert. Nach dem Andestillieren trägt man im Verlauf von 4 Stunden bei einer Reaktionstemperatur von 60°C und einem Arbeitsdruck von 250 mbar eine Suspension von 375 g Oxalsäure in 375 g Phosphoroxichlorid ein. Die Ausbeute an Rohdestillat beträgt 2052 g 21,9 gew.%iges Oxalylchlorid, aus dem durch Rektifikation 445 g Oxalylchlorid isoliert werden.

Beispiel 21

In einer Apparatur gemäss Beispiel 19 werden 1500 g Phosphoroxichlorid, 100 g 2,4-Dimethylpyridin und 1980 g Phosphortrichlorid vorgelegt. Dann leitet man 1040 g Chlor in ca. 2 Stunden ein, wobei man den Ansatz bei einer Sumpftemperatur von ca. 124°C am Rückfluss sieden lässt. Man destilliert den Überschuss Chlor ab, kühlt auf 60°C ab und trägt unter Rühren bei einer Reaktionstemperatur von 60°C und einem Arbeitsdruck von 240 mbar innerhalb von 4 Stunden 420 g wasserfreie Oxalsäure ein. Man isoliert 1813 g 26,8 gew.-%iges Oxalylchlorid-Rohdestillat, aus dem durch Rektifikation 481 g Oxalylchlorid gewonnen werden.

Die im Reaktor verbliebene Suspension kann nach Zusatz von 1215 g Phosphortrichlorid in gleicher Weise bei Siedetemperatur mit 640 g Chlor behandelt und anschliessend bei 60°C/240 mbar wie zuvor mit Oxalsäure versetzt werden.

**Patentansprüche**

1. Verfahren zur Herstellung von Oxalylchlorid aus Oxalsäureverbindungen der Formel

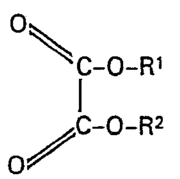

worin $R^1$ und $R^2$ gleich oder verschieden sind und für Wasserstoff oder einen niederen Alkylrest stehen, und Phosphorpentachlorid in Gegenwart von Phosphoroxichlorid, dadurch gekennzeichnet, dass man die Umsetzung in Gegenwart einer Aminoverbindung der Formel

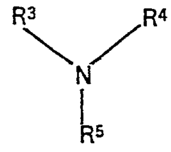

worin $R^3$ für gegebenenfalls durch Amino oder Carbonamido substituiertes Alkyl, Aralkyl, Aryl oder eine Acylgruppe steht und $R^4$ oder $R^5$ gleich oder verschieden sind und für Wasserstoff oder, gegebenenfalls durch Amino oder Carbonamido substituiertes, Alkyl, Aralkyl oder Aryl stehen, oder $R^4$ und $R^5$ in einem gegebenenfalls substituierten, gegebenenfalls Stickstoff, Schwefel und/oder Sauerstoff enthaltenden, und gegebenenfalls durch Alkyl-, Aralkyl-, Aryl- und/oder Aminogruppen substituierten carbocyclischen Ring mit 5 bis 7 Ringgliedern verbunden sein können und $R^3$ für Wasserstoff, für gegebenenfalls durch eine Amino- oder eine Carbonamidogruppe substituiertes Alkyl, das mit $R^4$ zu einem carbocyclischen Ring verbunden sein kann, Aralkyl oder Aryl steht oder gegebenenfalls in einem der Reste $R^4$ oder $R^5$ eine Doppelbindung bildet und gegebenenfalls in Gegenwart eines inerten Verdünnungsmittels durchführt und während der Reaktion das entstehende Oxalylchlorid abdestilliert.

2. Verfahren nach Anspruch 1, dadurch gekennzeichnet, dass man Aminoverbindungen der Formel

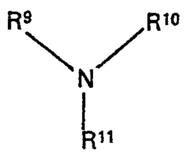

worin $R^9$ für gegebenenfalls durch eine Amino- oder eine Carbonamidogruppe substituiertes Alkyl mit 1 bis 4 Kohlenstoffatomen, Aralkyl mit 7 bis 9 Kohlenstoffatomen, Phenyl oder Acyl mit 1 bis 8 Kohlenstoffatomen steht, $R^{10}$ und $R^1$ gleich oder verschieden sind für gegebenenfalls durch Amino- oder Carbonamido substituiertes Alkyl mit 1 bis 4 Kohlenstoffatomen, Aralkyl mit 7 bis 9 Kohlenstoffatomen und Phenyl stehen, oder $R^{10}$ und $R^{11}$ in einem 5- oder 6-gliedrigen Ring, der gegebenenfals auch ein Stickstoff-, Schwefel oder ein Sauerstoffatom als weiteres Heteroatom enthält und gegebenenfalls durch 1-5 Alkylgruppen mit 1-4 Kohlenstoffatomen, einem ankondensierten, gegebenenfalls weiter substituierten Benzoring oder 1-2 Dialkylamino- oder carbocyclische Alkylidenaminogruppen mit je 1-4 Kohlenstoffatomen substituiert ist, verbunden sein können und $R^9$ für Wasserstoff, für gegebenenfalls durch eine Amino- oder eine Carbonamidogruppe substituiertes Alkyl mit 1 bis 4 Kohlenstoffatomen das mit $R^{10}$ zu einem carbocyclischen Ring verbunden sein kann, Aralkyl mit 7 bis 9 Kohlenstoffatomen, Phenyl oder Acyl mit 1 bis 8 Kohlenstoffatomen steht oder gegebenenfalls zu einem der Reste $R^{10}$ oder $R^{11}$ eine Doppelbindung bildet.

3. Verfahren nach Ansprüchen 1 und 2, dadurch gekenzeichnet, dass man als Aminoverbindungen Trimethylamin, Triäthylamin, Pyridin, 2-Methylpyridin, 4-Methylpyridin, 4-Äthylpyridin, 2,4-Dimethylpyridin, 1,5-Diazabicyclo-[4,3,0]-non-5-en, 1,8-Diazabicyclo-[5,4,0]-undec-7-en, Dimethylpiperazin, Chinolin und Isochinolin verwendet.

**Patent Claims**

1. Process for the preparation of oxalyl chloride from oxalic acid compounds of the formula

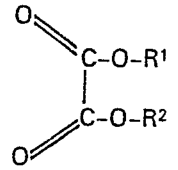

wherein $R^1$ and $R^2$ are identical or different and represent hydrogen or a lower alkyl radical, and phosphorus pentachloride in the presence of phosphorus oxychloride, characterised in that the reaction is carried out in the presence of an amino compound of the formula

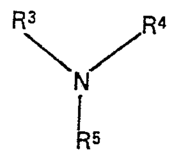

wherein $R^3$ represents alkyl, aralkyl, aryl or an acyl group, optionally substituted by amino or carboxamido and $R^4$ and $R^5$ are identical or different and represent hydrogen or alkyl, aralkyl, or aryl, optionally substituted by amino or carboxamido, or $R^4$ and $R^5$ can be linked in an optionally substituted carbocyclic ring with 5 to 7 ring members, which optionally contains nitrogen, sulphur and/or oxygen and is optionally substituted by alkyl, aralkyl, aryl and/or amino groups, and $R^3$ represents hydrogen or alkyl, which can be linked with $R^4$ to form a carbocyclic ring, aralkyl or aryl, optionally substituted by an amino or carboxamido group, or optionally forms a double bond in one of the radicals $R^4$ or $R^5$, and if appropriate in the presence of an inert diluent, and the oxalyl chloride formed is distilled off during the reaction.

2. Process according to Claim 1, characterised in that amino compounds of the formula

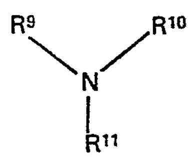

wherein $R^9$ represents alkyl with 1 to 4 carbon atoms, aralkyl with 7 to 9 carbon atoms, phenyl or acyl with 1 to 8 carbon atoms, optionally substituted by an amino or carboxamido group, and $R^{10}$ and $R^{11}$ are identical or different and represent alkyl with 1 to 4 carbon atoms, aralkyl with 7 to 9 carbon atoms and phenyl, optionally substituted by amino- or carboxamido, or $R^{10}$ and $R^{11}$ can be linked in a 5-membered or 6-membered ring, which optionally also contains a nitrogen, sulphur or oxygen atom as a further hetero-atom and optionally substituted by 1–5 alkyl groups with 1–4 carbon atoms, a fused-on, optionally further substituted benzo ring or 1–2 dialkylamino or carbocyclic alkylideneamino groups with 1–4 carbon atoms in each case, and $R^9$ represents hydrogen or alkyl with 1 to 4 carbon atoms, which can be linked with $R^{10}$ to form a carbocyclic ring, aralkyl with 7 to 9 carbon atoms, phenyl or acyl with 1 to 8 carbon atoms, optionally substituted by an amino or carboxamido group, or optionally forms a double bond to one of the radicals $R^{10}$ or $R^{11}$.

3. Process according to Claims 1 and 2, characterised in that trimethylamine, triethylamine, pyridine, 2-methylpyridine, 4-methylpyridine, 4-ethylpyridine, 2,4-dimethylpyridine, 1,5-diaza-bicyclo-[4,3,0]-non-5-ene, 1,8-diazabicyclo-[5,4,0]-undec-7-ene, dimethylpiperazine, quinoline and isoquinoline are used as the amino compounds.

**Revendications**

1. Procédé de préparation du chlorure d'oxalyle à partir de l'acide oxalique et de dérivés de l'acide oxalique répondant à la formule

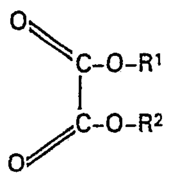

dans laquelle $R^1$ et $R^2$, identiques ou différents, représentent l'hydrogène ou un reste alkyle inférieur, et du pentachlorure de phosphore en présence d'oxychlorure de phosphore, caractérisé en ce que l'on effectue la réaction en présence d'un composé aminé répondant à la formule

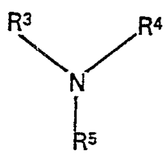

dans laquelle $R^3$ représente un groupe alkyle, aralkyle, aryle portant éventuellement des substituants amino ou carboxamido, ou un groupe acyle, et $R^4$ ou $R^5$, identiques ou différents, représentent l'hydrogène ou un groupe alkyle, aralkyle ou aryle portant éventuellement des substituants amino ou carboxamido, ou bien $R^4$ et $R^5$ peuvent être reliés avec formation d'un noyau carbocyclique de 5 à 7 chaînons cycliques éventuellement substitué, contenant éventuellement de l'azote, du soufre et/ou de l'oxygène, et substitué le cas échéant par des groupes alkyle, aralkyle, aryle et/ou amino, et $R^3$ représente l'hydrogène, un groupe alkyle portant éventuellement un substituant amino ou carboxamido, qui peut être relié à $R^4$ avec formation d'un noyau carbocyclique, un groupe aralkyle ou aryle, ou forme le cas échéant une double liaison dans l'un des restes $R^4$ ou $R^5$, et le cas échéant en présence d'un diluant inerte, en distillant le chlorure d'oxalyle formé en cours de réaction.

2. Procédé selon la revendication 1, caractérisé en ce que l'on utilise des composés aminés de formule

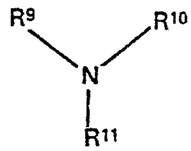

dans laquelle $R^9$ représente un groupe alkyle en $C_1$–$C_4$, aralkyle en $C_7$–$C_9$, phényle ou acyle en $C_1$–$C_8$ portant éventuellement un substituant amino ou carboxamido, $R^{10}$ et $R^{11}$, identiques ou différents, représentent un groupe alkyle en $C_1$–$C_4$, aralkyle en $C_7$–$C_9$ et phényle portant éventuellement des substituants amino ou carboxamido, ou bien $R^{10}$ et $R^{11}$ peuvent être reliés avec formation d'un noyau à 5 ou 6 chaînons qui peut contenir également le cas échéant un atome d'azote, de soufre ou d'oxygène comme autre hétéroatome et est substitué le cas échéant par 1 à 5 groupes alkyle en $C_1$–$C_4$, un noyau benzénique condensé portant éventuellement d'autres substituants, ou 1 ou 2 groupes dialkylamino ou alkylidène-amino carbocyclique contenant chacun 1 à 4 atomes de carbone, et $R^9$ représente l'hydrogène, un groupe alkyle en $C_1$–$C_4$ portant éventuellement un substituant amino ou carboxamido et qui peut être relié à $R^{10}$ avec formation d'un noyau carbocyclique, un groupe aralkyle en $C_7$–$C_9$, phényle ou acyle en $C_1$–$C_8$, ou forme le cas échéant une double liaison avec l'un des restes $R^{10}$ ou $R^{11}$.

3. Procédé selon les revendications 1 et 2, caractérisé en ce que l'on utilise en tant que composés aminés la triméthylamine, la triéthylamine, la pyridine, la 2-méthyl-pyridine, la 4-méthyl-pyridine, la 4-éthyl-pyridine, la 2,4-diméthyl-pyridine, le 1,5-diazabicyclo-[4,3,0]-nona-5-ène, le 1,8-diazabicyclo-[5,4,0]-undéca-7-ène, la diméthyl-pipérazine, la quinoléine et l'isoquinoléine.